# FASCICULE DE BREVET EUROPEEN

(11) **EP 3 250 269 B1**
(45) Date de publication et mention de la délivrance du brevet: **10.08.2022**
(21) Numéro de dépôt: 16707843.5
(22) Date de dépôt: 28.01.2016
(51) Int. Cl.: A61M 5/315

(54) **BOUCHON-PISTON DE SERINGUE**
SPRITZENKOLBENSTOPPER
SYRINGE PLUNGER-STOPPER

(30) Priorité: 29.01.2015 FR 1550678
(43) Date de publication de la demande: 06.12.2017
(73) Titulaire: Aptar Stelmi SAS, 93420 Villepinte (FR)
(72) Inventeur: FOURNIER, Ghislain, 17000 La Rochelle (FR); SWAL, Mickaël, 77124 Chauconin Neufmontiers (FR)
(74) Mandataire: CAPRI
(86) Numéro de dépôt international: PCT/FR2016/050173
(87) Numéro de publication internationale: WO 2016/120565

(56) Documents cités:
- EP-A1- 2 803 378
- EP-A2- 1 002 551
- WO-A1-01/60434
- WO-A1-2012/101669
- JP-A- 2001 190 667
- US-A- 5 735 825
- US-A- 6 004 300
- US-A1- 2007 219 508
- US-A1- 2011 077 601
- US-A1- 2014 062 036

## Description

La présente invention concerne un bouchon-piston de seringue.

Le bouchon-piston pour seringue est bien connu dans l'état de la technique. Avant actionnement de la seringue, il remplit la fonction de bouchon en isolant le produit fluide contenu dans le corps de seringue, et lors de l'actionnement il se transforme en piston en poussant le produit fluide hors du corps de seringue, généralement à travers une aiguille. Généralement, le dispositif de seringue comporte un organe d'actionnement, tel qu'une tige de piston, qui coopère avec ledit bouchon-piston pour le déplacer dans le corps de seringue lors de l'actionnement. Ce bouchon-piston doit donc assurer l'étanchéité et est donc généralement réalisé en caoutchouc ou autre élastomère similaire. Un inconvénient avec ce type de matériau concerne les risques d'interactions entre le matériau du bouchon-piston et le produit fluide à distribuer, notamment pendant la phase de stockage, ces interactions pouvant altérer ledit produit fluide. Pour limiter ces risques d'interactions, il a été proposé de revêtir la surface frontale du bouchon-piston, c'est-à-dire la surface en contact avec le produit fluide pendant le stockage et pendant la distribution du produit, avec un revêtement approprié. Il a ainsi notamment été proposé de disposer un film mince en ETFE (éthylène tétrafluoroéthylène) sur la surface frontale du bouchon-piston. Cette mise en oeuvre permet de limiter les risques d'interactions entre le matériau du bouchon-piston et le produit fluide. L'application dudit film rend toutefois le procédé de fabrication plus complexe, et impose une zone de découpe du bouchon-piston après moulage qui n'est pas optimale. De plus, la présence de ce revêtement sur la surface frontale rigidifie celle-ci, et peut donc rendre l'assemblage et le déplacement du bouchon-piston dans le corps de seringue plus difficile. Plus précisément, la présence de ce revêtement frontal diminue la capacité de déformation de la surface frontale du bouchon-piston et peut donc rendre son insertion et son glissement dans le corps de seringue plus difficile. Il se pose donc le problème de trouver le bon compromis entre une étanchéité suffisante pour le bouchon-piston une fois inséré dans le corps de seringue et un processus d'assemblage et d'actionnement qui ne soit pas trop compliqué ou difficile.

Par ailleurs, la coopération entre le bouchon-piston et l'organe d'actionnement, en général une tige de piston, peut se faire généralement par l'intermédiaire d'un filetage prévu sur la surface interne du bouchon-piston et qui coopère avec un filet correspondant aménagé sur l'extrémité avant de la tige de piston. Cette mise en œuvre complique également la fabrication du bouchon piston, notamment la phase de démoulage, en raison des contre-dépouilles importantes du moule de fabrication du bouchon piston nécessaires pour mouler le filetage.

Les documents WO2006021380, EP1180377, EP1674121, DE10006560, WO02092312, JP2001190667, US2007219508, WO2012101669, EP1002551, US2014062036, US5735825 et EP2803378 décrivent des dispositifs de l'état de la technique.

Le document WO2015052429 décrit un bouchon-piston de seringue similaire à celui de la présente invention.

La présente invention a pour but de fournir un bouchon-piston qui ne reproduit pas les inconvénients susmentionnés.

En particulier, la présente invention a pour but de fournir un bouchon-piston qui garantit une parfaite étanchéité lors du stockage et lors de l'actionnement, tout en permettant un procédé de fabrication, un procédé d'assemblage et un actionnement simplifié et fiable.

La présente invention a également pour but de fournir un bouchon-piston qui soit plus simple et donc moins coûteux à fabriquer et à assembler, notamment dans le processus de moulage dudit bouchon-piston.

La présente invention a donc pour objet un bouchon-piston comportant un corps cylindrique ayant un côté axial avant fermé par une paroi frontale, ledit bouchon-piston comportant une surface externe pourvue d'au moins un profil d'étanchéité secondaire, ladite paroi frontale comportant une surface externe axiale frontale pourvue d'un revêtement, avantageusement un film en éthylène tétrafluoroéthylène, ladite paroi frontale comportant sur son bord radialement externe un profil d'étanchéité primaire, ladite paroi frontale et ledit corps cylindrique étant réalisés avec des matériaux différents, ledit corps cylindrique creux étant surmoulé sur ladite paroi frontale, ladite surface externe axiale frontale de ladite paroi frontale comportant un profil de déformation disposé radialement à l'intérieur dudit profil d'étanchéité primaire de ladite paroi frontale, ledit profil de déformation étant adapté à se déformer radialement pour faciliter l'insertion et/ou le glissement dudit bouchon-piston dans un corps de seringue.

Avantageusement, ledit corps cylindrique est réalisé en un matériau caoutchouc, comprenant par un ou plusieurs élastomères synthétiques polymères, tels que le caoutchouc butyle, le caoutchouc chlorobutyl, le caoutchouc bromobutyl, des copolymères de caoutchouc d'isobutylène et de par-méthylstyrène, le caoutchouc isoprène, le SBR caoutchouc, le NBR, l'EPDM.

Avantageusement, ledit matériau est associé à au moins un accélérateur et/ou charge.

Avantageusement, ladite paroi frontale est réalisée en un matériau caoutchouc, comprenant par un ou plusieurs élastomères synthétiques polymères, tels que le caoutchouc butyle, le caoutchouc chlorobutyl, le caoutchouc bromobutyl, des copolymères de caoutchouc d'isobutylène et de par-méthylstyrène, le caoutchouc isoprène, le SBR caoutchouc, le NBR, l'EPDM.

Avantageusement, ledit matériau est associé à au moins un accélérateur et/ou charge.

Avantageusement, ledit au moins un accélérateur et/ou charge dudit corps cylindrique est différent, en quantité et/ou en composition, dudit au moins un accélérateur et/ou charge de ladite paroi frontale.

Avantageusement, ledit corps cylindrique comporte un profil d'étanchéité secondaire et ladite paroi frontale comporte un profil d'étanchéité secondaire.

Avantageusement, lesdits profils d'étanchéité secondaires sont des bourrelets radialement saillants.

Avantageusement, ledit profil de déformation est réalisé sous la forme d'une rainure périphérique ménagée dans ladite surface externe axiale frontale de ladite paroi frontale, ladite rainure étant avantageusement en forme de V ou de U.

Avantageusement, ledit corps cylindrique est creux et définit un volume interne, ledit corps cylindrique creux ayant un côté axial arrière ouvert axialement opposé audit côté axial avant fermé par ladite paroi frontale.

Avantageusement, ledit volume interne coopère avec un organe d'actionnement d'un dispositif de seringue.

Avantageusement, ledit corps cylindrique creux comporte sur sa surface interne un filetage adapté à se visser sur un filet dudit organe d'actionnement.

Avantageusement, ladite surface externe axiale frontale est conique et définit une pointe axiale centrale.

Avantageusement, ledit profil de déformation est de forme arrondie, de sorte que ladite surface externe axiale frontale de ladite paroi frontale est dépourvue d'angles vifs.

Ces caractéristiques et avantages et d'autres de la présente invention apparaîtront plus clairement au cours de la description détaillée suivante, faite en référence aux dessins joints, donnés à titre d'exemples non-limitatifs, et sur lesquels :
La figure 1 est une vue schématique en section transversale d'un bouchon-piston selon l'état de la technique,
La figure 2 est une vue similaire à celle de la figure 1, illustrant un bouchon-piston selon un mode de réalisation avantageux de la présente invention,
Les figures 3 et 4 illustrent schématiquement l'étape de moulage du procédé de fabrication selon l'état de la technique du bouchon-piston de la figure 1, respectivement avec le moule fermé et ouvert,
La figure 5 illustre schématiquement l'étape de découpage du procédé de fabrication selon l'état de la technique du bouchon-piston de la figure 1, et Les figures 6 à 11 illustrent schématiquement les étapes de moulage et de découpage du procédé de fabrication selon un mode de réalisation avantageux de l'invention du bouchon-piston de la figure 2.

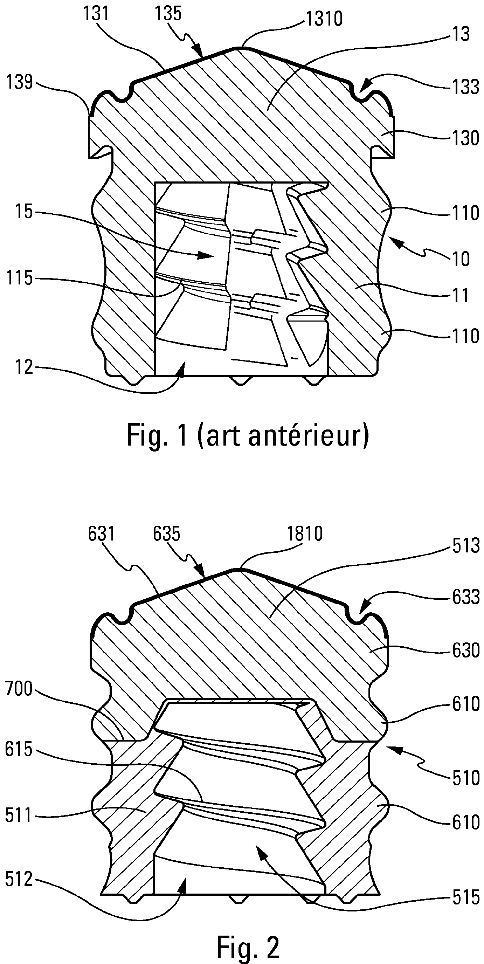

La figure 1 représente un bouchon-piston selon l'état de la technique, notamment tel que décrit dans la demande de brevet co-pendante WO2015052429.

Le bouchon-piston 10, moulé d'une pièce en élastomère, en caoutchouc ou en tout autre matériau approprié, comporte un corps cylindrique 11, de préférence creux. Ce corps cylindrique creux comporte un côté axial arrière ouvert 12 et un côté axial avant qui est fermé par une paroi frontale 13. Ainsi, cette mise en œuvre définit un bouchon-piston 10 de forme généralement cylindrique avec un volume interne 15 borgne, c'est-à-dire ouvert d'un côté et fermé de l'autre côté.

La paroi frontale 13 définit une surface externe axiale frontale 131 qui est la surface qui sera en contact du produit fluide lorsque le bouchon-piston 10 sera assemblé dans un corps de seringue (non représenté) destiné à contenir un tel produit fluide. Cette surface externe axiale frontale 131 est pourvue d'un revêtement 135, avantageusement un film mince réalisé en ETFE (éthylène tétrafluoroéthylène), afin de limiter au maximum les interactions entre ledit produit fluide et le matériau dudit bouchon-piston. D'autres types de revêtements appropriés sont aussi envisageables, aussi bien en ce qui concerne le mode d'application du revêtement que le matériau constituant ledit revêtement. Cette surface externe axiale frontale 131 peut être conique en définissant une pointe axiale 1310, comme visible sur le mode de réalisation de la figure 1, mais elle pourrait également être plane et sensiblement perpendiculaire à l'axe central du bouchon-piston.

La surface externe latérale du corps cylindrique creux 11 comporte au moins un, de préférence plusieurs profils d'étanchéité 110, qui sont généralement réalisés sous la forme de bourrelets radialement saillants. De préférence, comme représenté sur la figure 1, la surface externe du corps cylindrique creux 11 comporte deux profils d'étanchéité 110 réalisés sous la forme de bourrelets radialement saillants. La paroi frontale 13 peut également comporter un profil d'étanchéité 130 réalisé sur son bord radialement externe.

Ladite surface externe axiale frontale 131 de ladite paroi frontale 13 comporte un profil de déformation 133 qui est adapté à se déformer pour faciliter l'insertion du bouchon-piston 10 dans un corps de seringue. Ce profil de déformation peut être disposé radialement à l'intérieur dudit profil d'étanchéité 130, c'est-à-dire qu'il n'est pas nécessairement disposé au niveau du bord radialement externe de ladite paroi frontale 13. Avantageusement, ce profil de déformation 133 peut être réalisé sous la forme d'une rainure périphérique ménagée dans ladite surface externe axiale frontale 131. Dans l'exemple représenté sur la figure 1, cette rainure, avant déformation, est sensiblement en forme de U, mais elle pourrait évidemment également avoir une autre forme, par exemple une forme en V, en W ou toute autre forme appropriée permettant une déformation radiale de la paroi frontale 13 lors de l'insertion du bouchon-piston.

De manière avantageuse, ledit profil de déformation 133 est de forme arrondie, c'est-à-dire qu'il ne définit pas d'angle vif dans ladite surface externe axiale frontale 131 de ladite paroi frontale 13. Or, de tels angles vifs peuvent générer des zones de faiblesse du revêtement 135, non seulement pendant le moulage du bouchon-piston où ils peuvent être générateur d'arrachée, mais aussi en utilisation, où le revêtement 135 pourrait se fissurer ou se déchirer au niveau desdits angles vifs, par exemple lors de la compression du bouchon-piston pendant sa mise en place dans la seringue, avec par conséquent des risques de discontinuité dudit revêtement.

Avantageusement, ledit revêtement 135 s'étend non seulement sur ladite surface externe axiale frontale 131, mais également au moins partiellement sur la surface cylindrique externe dudit profil d'étanchéité 130. Ceci permet de garantir la présence d'une première zone d'étanchéité cylindrique entre l'intérieur d'un corps de seringue et la partie revêtue dudit profil d'étanchéité 130 du bouchon-piston 10. Ceci améliore en particulier l'étanchéité lors de fortes pressions exercées dans la seringue. En effet, une zone de contact cylindrique entre le corps de seringue et la zone revêtue du bouchon-piston 10 garantit l'absence de micro-pliures du revêtement 135 et permet ainsi d'avoir une zone d'étanchéité de contact constante entre le revêtement 135 du bouchon-piston et le corps de seringue.

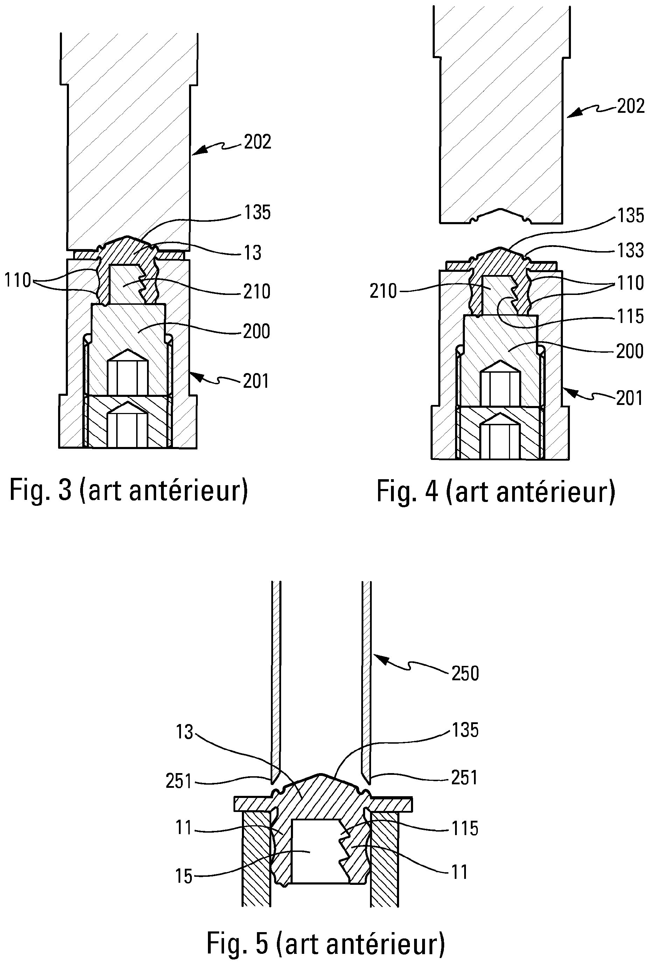

En particulier, ce revêtement 135 s'étend jusqu'à un épaulement 139 dudit profil d'étanchéité 130. Cet épaulement 139 est généré par le processus de fabrication du bouchon-piston de la figure 1, comme cela sera décrit plus en détails ci-après en référence aux figures 3 à 5.

Pendant l'insertion du bouchon-piston 10 dans un corps de seringue, le profil de déformation 133 peut se déformer radialement, ce qui permet au profil d'étanchéité 130 de la paroi frontale 13 de pénétrer plus facilement à l'intérieur du corps de seringue.

Après insertion dudit bouchon-piston 10 dans le corps de seringue, les différents profils d'étanchéité, c'est-à-dire le profil d'étanchéité 130 de la paroi frontale 13 et les profils d'étanchéité 110 du corps cylindrique creux 11 peuvent légèrement se détendre tout en restant suffisamment contraints contre la paroi cylindrique du corps de seringue afin d'assurer une parfaite étanchéité.

La présence du profil de déformation 133 dans la surface externe axiale frontale 131 de la paroi frontale 13 facilite cette insertion. En effet, la présence du revêtement 135 rigidifie le matériau et rend donc généralement son insertion plus compliquée. Le fait de prévoir un profil de déformation permet de compenser l'augmentation de frottement liée au revêtement ETFE, et ainsi permet d'assurer une insertion plus fiable et sûre du bouchon-piston revêtu tel que décrit ci-dessus. De même, la présence du profil de déformation 133 dans la surface externe axiale frontale 131 de la paroi frontale 13 du piston avec revêtement permet d'avoir un comportement identique à celui d'un piston sans revêtement, notamment en ce qui concerne les forces d'activation et de glissement du piston lors de l'actionnement.

Le volume interne 15 du bouchon-piston 10 peut comporter un filetage 115. Ce filetage 115 peut être continu ou discontinu. Dans la variante d'un filetage discontinu, plusieurs rainures axiales, avantageusement quatre, peuvent être prévues pour interrompre ledit filetage 115.

Les figures 3 à 5 illustrent une machine de fabrication du bouchon-piston de la figure 1, avec une première partie de moule 201 qui est la partie inférieure sur les figures 3 et 4 et une seconde partie du moule 202 qui est la partie supérieure. La première partie du moule 201 est utilisée pour former le corps cylindrique creux 11 et comporte à cet effet un noyau 200. Ce noyau 200 comporte un embout axial 210 qui va définir ledit volume interne 15 dudit bouchon-piston 10. Ce procédé de fabrication du bouchon-piston de la figure 1 est complexe car il nécessite plusieurs contre-dépouilles sur la première partie de moule 201 et sur l'embout axial 210 du noyau 200, pour former d'une part le filetage interne 115 et d'autre part les profils d'étanchéité 110. Ceci rend le démoulage plus difficile.

La figure 5 illustre l'étape de découpage du bouchon-piston. On constate qu'en raison du revêtement 135, la découpe ne peut pas être réalisée avec tension de la toile, ce qui engendre un diamètre de découpe légèrement supérieur à celui des profils d'étanchéité 110 moulés, et donc ledit épaulement 139 visible sur la figure 1. Ainsi, la première zone d'étanchéité formée par le profil d'étanchéité 130 est réalisée au niveau de la zone de découpe, ce qui génère un état de surface non lisse, avec par conséquent des risques potentiels d'infiltrations et/ou de glissement du piston. En effet, un piston ayant comme première zone d'étanchéité une zone de découpe comportant des micros-stries (dues à la découpe) provoque :
- une moins bonne performance à l'étanchéité (micro-passages pour le liquide par les stries), et
- un moins bon glissement, du fait de l'état de surface et du diamètre de découpe plus important que les profils d'étanchéité 110 réalisés par moulage, car cette zone de découpe ne peut être réalisée avec tension, en raison de la rigidité du revêtement 135.

De manière connue, le bouchon-piston 10 peut en outre être revêtu en totalité d'huile de silicone pour favoriser son glissement, notamment lors de son insertion dans la seringue. En variante, si l'on souhaite supprimer cette huile de silicone, on peut aussi prévoir un revêtement parylène, également sur la totalité du bouchon-piston. Dans ce cas, le film ETFE sur la surface frontale du piston pourra être traité afin de permettre l'accroche du parylène.

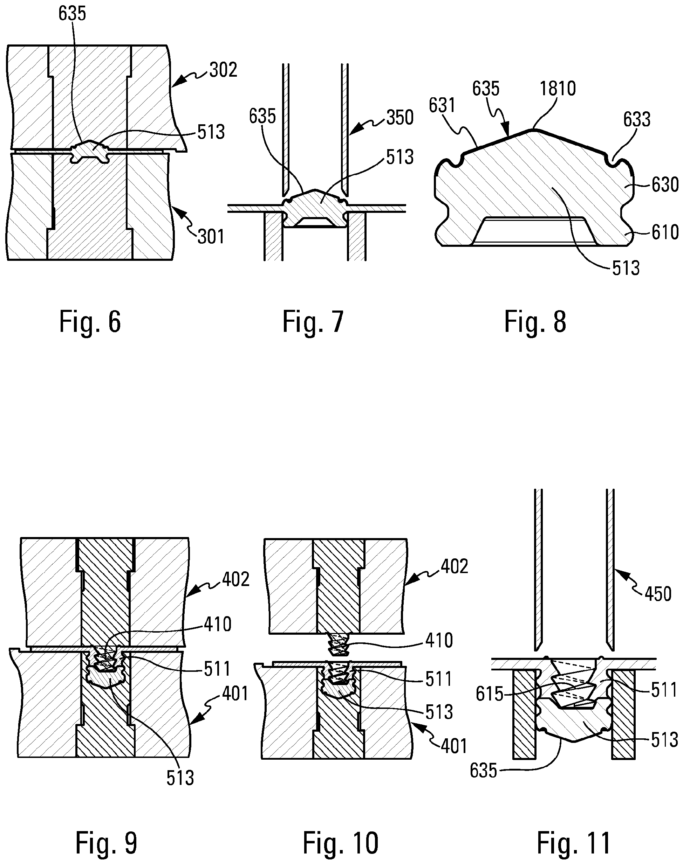

Selon l'invention, le bouchon-piston est réalisé par surmoulage de deux parties. La figure 2 illustre un tel bouchon-piston, et les figures 6 à 11 illustrent un procédé de fabrication dudit bouchon-piston.

Dans des buts de clarté, les éléments dudit bouchon-piston et dudit procédé selon l'invention qui sont similaires à ceux de l'art antérieur représenté sur les figures 1 et 3 à 5 comportent les mêmes références numériques augmentées de 500.

Ainsi, selon l'invention, le bouchon-piston 510 comporte une première partie moulée formée d'une paroi frontale 513 et une seconde partie surmoulée sur ladite première partie moulée formée d'un corps cylindrique 511, de préférence creux. La ligne de séparation 700 illustre cette constitution en deux parties.

Si le corps cylindrique 511 est creux, il comporte un côté axial arrière ouvert 512 et un côté axial avant qui est fermé par ladite paroi frontale 513. Ainsi, cette mise en œuvre définit un bouchon-piston 510 de forme généralement cylindrique avec un volume interne 515 borgne, c'est-à-dire ouvert d'un côté et fermé de l'autre côté. La paroi frontale 513 définit une surface externe axiale frontale 631 qui est la surface qui sera en contact du produit fluide lorsque le bouchon-piston 510 sera assemblé dans un corps de seringue (non représenté) destiné à contenir un tel produit fluide. Cette surface externe axiale frontale 631 est pourvue d'un revêtement 635, avantageusement un film mince réalisé en ETFE (éthylène tétrafluoroéthylène), afin de limiter au maximum les interactions entre ledit produit fluide et le matériau dudit bouchon-piston. D'autres types de revêtements appropriés sont aussi envisageables, aussi bien en ce qui concerne le mode d'application du revêtement que le matériau constituant ledit revêtement. Cette surface externe axiale frontale 631 peut être conique en définissant une pointe axiale 1810, comme visible sur le mode de réalisation de la figure 2, mais elle pourrait également être plane et sensiblement perpendiculaire à l'axe central du bouchon-piston.

La paroi frontale 513 comporte un profil d'étanchéité primaire 630 réalisé sur son bord radialement externe. La surface externe du bouchon-piston 510 comporte en outre au moins un, de préférence plusieurs profils d'étanchéité secondaires 610, qui sont généralement réalisés sous la forme de bourrelets radialement saillants. De préférence, comme représenté sur la figure 2, la surface externe du bouchon-piston 510 comporte deux profils d'étanchéité secondaires 610 réalisés sous la forme de bourrelets radialement saillants. Dans l'exemple représenté sur la figure 2, l'un des profils d'étanchéité secondaires 610 est formé sur ladite partie frontale 513 et l'autre sur ledit corps cylindrique creux 511, mais en variante les deux profils d'étanchéité secondaires 610 pourraient être formés sur ledit corps cylindrique creux 511.

Ladite surface externe axiale frontale 631 de ladite paroi frontale 513 comporte un profil de déformation 633 qui est adapté à se déformer pour faciliter l'insertion du bouchon-piston 510 dans un corps de seringue. Ce profil de déformation peut être disposé radialement à l'intérieur dudit profil d'étanchéité primaire 630, c'est-à-dire qu'il n'est pas nécessairement disposé au niveau du bord radialement externe de ladite paroi frontale 513. Avantageusement, ce profil de déformation 633 peut être réalisé sous la forme d'une rainure périphérique ménagée dans ladite surface externe axiale frontale 631. Dans l'exemple représenté sur la figure 2, cette rainure, avant déformation, est sensiblement en forme de U, mais elle pourrait évidemment également avoir une autre forme, par exemple une forme en V, en W ou toute autre forme appropriée permettant une déformation radiale de la paroi frontale 513 lors de l'insertion du bouchon-piston.

De manière avantageuse, ledit profil de déformation 633 est de forme arrondie, c'est-à-dire qu'il ne définit pas d'angle vif dans ladite surface externe axiale frontale 631 de ladite paroi frontale 513. Or, de tels angles vifs peuvent générer des zones de faiblesse du revêtement 635, non seulement pendant le moulage du bouchon-piston où ils peuvent être générateur d'arrachée, mais aussi en utilisation, où le revêtement 635 pourrait se fissurer ou se déchirer au niveau desdits angles vifs, par exemple lors de la compression du bouchon-piston pendant sa mise en place dans la seringue, avec par conséquent des risques de discontinuité dudit revêtement.

Avantageusement, ledit revêtement 635 s'étend non seulement sur ladite surface externe axiale frontale 631, mais également au moins partiellement sur la surface cylindrique externe dudit profil d'étanchéité 630. Ceci permet de garantir la présence d'une première zone d'étanchéité cylindrique entre l'intérieur d'un corps de seringue et la partie revêtue dudit profil d'étanchéité primaire 630 du bouchon-piston 510. Ceci améliore en particulier l'étanchéité lors de fortes pressions exercées dans la seringue. En effet, une zone de contact cylindrique entre le corps de seringue et la zone revêtue du bouchon-piston 510 garantit l'absence de micro-pliures du revêtement 635 et permet ainsi d'avoir une zone d'étanchéité de contact constante entre le revêtement 635 du bouchon-piston et le corps de seringue.

Pendant l'insertion du bouchon-piston 510 dans un corps de seringue, le profil de déformation 633 peut se déformer radialement, ce qui permet au profil d'étanchéité primaire 630 de la paroi frontale 513 de pénétrer plus facilement à l'intérieur du corps de seringue.

Après insertion dudit bouchon-piston 510 dans le corps de seringue, les différents profils d'étanchéité, c'est-à-dire le profil d'étanchéité primaire 630 et les profils d'étanchéité secondaires 610 peuvent légèrement se détendre tout en restant suffisamment contraints contre la paroi cylindrique du corps de seringue afin d'assurer une parfaite étanchéité.

La présence du profil de déformation 633 dans la surface externe axiale frontale 631 de la paroi frontale 513 facilite cette insertion. En effet, la présence du revêtement 635 rigidifie le matériau et rend donc généralement son insertion plus compliquée. Le fait de prévoir un profil de déformation permet de compenser l'augmentation de frottement liée au revêtement ETFE, et ainsi permet d'assurer une insertion plus fiable et sûre du bouchon-piston revêtu tel que décrit ci-dessus. De même, la présence du profil de déformation 633 dans la surface externe axiale frontale 631 de la paroi frontale 513 du piston avec revêtement permet d'avoir un comportement identique à celui d'un piston sans revêtement, notamment en ce qui concerne les forces d'activation et de glissement du piston lors de l'actionnement.

Le volume interne 515 du bouchon-piston 510 peut comporter un filetage 615. Ce filetage 615 peut être continu ou discontinu. Dans la variante d'un filetage discontinu, plusieurs rainures axiales, avantageusement quatre, peuvent être prévues pour interrompre ledit filetage 615. En variante, le bouchon-piston peut être plein, c'est-à-dire ne pas comporter de volume interne creux.

Le bouchon-piston 510 selon l'invention, réalisé en deux parties surmoulées, présente notamment l'avantage d'être formé à partir de deux matériaux différents, ayant des propriétés optimisées. Ainsi, des propriétés mécaniques (dureté, élasticité, allongement) permettent d'optimiser les propriétés fonctionnelles du piston (glissement, étanchéité, passage machine, etc...), et des propriétés chimiques des matériaux (niveau d'extractibles, etc...) permettent d'utiliser un matériau de haute pureté pour la paroi frontale 513 en contact avec le film (1^{ère} étape de moulage), et de le combiner à un autre matériau aux propriétés fonctionnelles optimisées (en 2^{ème} étape de moulage).

Avantageusement, le corps cylindrique 511 et la paroi frontale 513 sont chacun réalisé en un matériau caoutchouc, comprenant par exemple un ou plusieurs élastomères synthétiques polymères, tels que le caoutchouc butyle (copolymère d'isobutylène et d'isoprène), le caoutchouc chlorobutyl (copolymère d'isobutylène-isoprène dérivé de la réaction d'un caoutchouc butyle avec du chlore), le caoutchouc bromobutyl (copolymère d'isobutylène-isoprène dérivé de la réaction d'un caoutchouc butyle avec du brome), des copolymères de caoutchouc d'isobutylène et de par-méthylstyrène, le caoutchouc isoprène (polyisoprène synthétique), le SBR caoutchouc (copolymère de styrène-butadiène), le NBR (copolymère de butadiène acrylonitrile), le caoutchouc EPDM (terpolymère éthylène-propylène-diène). Ces matériaux peuvent être associés à différents accélérateurs et/ou charges permettant d'obtenir les caractéristiques voulues,

Selon l'invention, ledit corps cylindrique 511 et ladite paroi frontale 513 sont réalisés dans des matériaux différents. Les caoutchoucs peuvent être différents. En variante, le corps cylindrique 511 peut avantageusement être réalisé en un matériau pouvant être composé des mêmes bases de caoutchouc que celles de ladite paroi frontale 513, mais associées à une ou des charges et/ou accélérateurs différentes dans leurs quantités et/ou compositions.

Les figures 6 à 11 illustrent le procédé de fabrication du bouchon-piston de la figure 2, selon un mode de réalisation avantageux de la présente invention.

La figure 6 montre le moulage de la paroi frontale 513 ensemble avec le revêtement 635, réalisé dans un premier moule comportant classiquement. deux parties de moules 301, 302. Le démoulage est aisé, car ledit moule ne comporte aucune contre-dépouille.

La figure 7 illustre la découpe de ladite paroi frontale moulée, notamment au moyen d'un poinçon ou d'une matrice 350.

La figure 8 représente la paroi frontale 513 après moulage et découpe, et avant surmoulage du corps cylindrique creux 511.

Les figures 9 et 10 illustrent le surmoulage du corps cylindrique creux 511 sur la paroi frontale 513. La paroi frontale 513 est disposée dans une première partie de moule 401 d'un second moule et ledit corps cylindrique creux 511 est surmoulé autour d'un noyau 410 d'une seconde partie 402 dudit second moule. Ledit noyau 410 définit ledit volume interne 515, et il comporte donc avantageusement un filetage externe qui va définir le filetage interne 615 dudit bouchon-piston 510. Le démoulage est simplifié: extraction du noyau (par exemple par déformation du matériau moulé) par déplacement de la partie supérieure du moule, puis démoulage de la nappe (contre-dépouilles définissant les profils d'étanchéité secondaires 610 pouvant être démoulées par déformation radiale du bouchon-piston vers l'intérieur, grâce au vide laissé par l'extraction du noyau. Ainsi, lors de cette étape de surmoulage, la zone de découpe de ladite paroi frontale moulée, générée par l'étape de découpe représentée sur la figure 7, sera re-conformée dans ledit second moule, et de ce fait le diamètre de cette zone de découpe sera inférieur à celui découpé. Le fait de placer ladite paroi frontale moulée dans un second moule permet donc d'éviter un inconvénient de l'art antérieur tel que décrit précédemment.

La figure 11 représente l'étape finale de découpe. Ici, la découpe peut être réalisée de manière conventionnelle sur l'arrière du bouchon-piston, au niveau du côté axial arrière ouvert 512, comme lorsque le bouchon-piston ne comporte pas de revêtement 635 sur sa surface externe axiale frontale 631. Ceci permet d'avoir une toile tendue au moment de la découpe, générant ainsi un retrait radial de cette zone une fois coupée par rapport aux profils d'étanchéité secondaires 610. Cette étape de découpe est classiquement réalisée au moyen d'un poinçon ou d'une matrice 450.

De manière connue, le bouchon-piston 510 peut en outre être revêtu en totalité d'huile de silicone pour favoriser son glissement, notamment lors de son insertion dans la seringue. En variante, si l'on souhaite supprimer cette huile de silicone, on peut aussi prévoir un revêtement parylène, également sur la totalité du bouchon-piston. Dans ce cas, le film ETFE sur la surface frontale du piston pourra être traité afin de permettre l'accroche du parylène.

Bien que la présente invention ait été décrite en référence à un mode de réalisation particulier, il est entendu que la présente invention n'est pas limitée par celui-ci mais qu'au contraire l'homme du métier peut y apporter toutes modifications utiles sans sortir du cadre de la présente invention tel que défini par les revendications annexées.

## Revendications

1. Bouchon-piston (510) comportant un corps cylindrique (511) ayant un côté axial avant fermé par une paroi frontale (513), ledit bouchon-piston (510) comportant une surface externe pourvue d'au moins un profil d'étanchéité secondaire (610), ladite paroi frontale (513) comportant une surface externe axiale frontale (631) pourvue d'un revêtement (635), avantageusement un film en éthylène tétrafluoroéthylène, ladite paroi frontale (513) comportant sur son bord radialement externe un profil d'étanchéité primaire (630), **caractérisé en ce que** ladite paroi frontale (513) et ledit corps cylindrique (511) sont réalisés avec des matériaux différents, ledit corps cylindrique creux (511) étant surmoulé sur ladite paroi frontale (513), et **en ce que** ladite surface externe axiale frontale (631) de ladite paroi frontale (513) comporte un profil de déformation (633) disposé radialement à l'intérieur dudit profil d'étanchéité primaire (630) de ladite paroi frontale (513), ledit profil de déformation (633) étant adapté à se déformer radialement pour faciliter l'insertion et/ou le glissement dudit bouchon-piston (510) dans un corps de seringue.

2. Bouchon-piston selon la revendication 1, dans lequel ledit corps cylindrique (511) est réalisé en un matériau caoutchouc, comprenant par un ou plusieurs élastomères synthétiques polymères, tels que le caoutchouc butyle, le caoutchouc chlorobutyl, le caoutchouc bromobutyl, des copolymères de caoutchouc d'isobutylène et de par-méthylstyrène, le caoutchouc isoprène, le SBR caoutchouc, le NBR, l'EPDM.

3. Bouchon-piston selon la revendication 2, dans lequel ledit matériau est associé à au moins un accélérateur et/ou charge.

4. Bouchon-piston selon l'une quelconque des revendications précédentes, dans lequel ladite paroi frontale (513) est réalisée en un matériau caoutchouc, comprenant par un ou plusieurs élastomères synthétiques polymères, tels que le caoutchouc butyle, le caoutchouc chlorobutyl, le caoutchouc bromobutyl, des copolymères de caoutchouc d'isobutylène et de par-méthylstyrène, le caoutchouc isoprène, le SBR caoutchouc, le NBR, l'EPDM.

5. Bouchon-piston selon la revendication 4, dans lequel ledit matériau est associé à au moins un accélérateur et/ou charge.

6. Bouchon-piston selon les revendications 3 et 5, dans lequel ledit au moins un accélérateur et/ou charge dudit corps cylindrique (511) est différent, en quantité et/ou en composition, dudit au moins un accélérateur et/ou charge de ladite paroi frontale (513).

7. Bouchon-piston selon l'une quelconque des revendications précédentes, dans lequel ledit corps cylindrique (511) comporte un profil d'étanchéité secondaire (610) et ladite paroi frontale (513) comporte un profil d'étanchéité secondaire (610).

8. Bouchon-piston selon la revendication 7, dans lequel lesdits profils d'étanchéité secondaires (610) sont des bourrelets radialement saillants.

9. Bouchon-piston selon l'une quelconque des revendications précédentes, dans lequel ledit profil de déformation (633) est réalisé sous la forme d'une rainure périphérique ménagée dans ladite surface externe axiale frontale (631) de ladite paroi frontale (513), ladite rainure étant avantageusement en forme de V ou de U.

10. Bouchon-piston selon l'une quelconque des revendications précédentes, dans lequel ledit corps cylindrique (511) est creux et définit un volume interne (515), ledit corps cylindrique creux (511) ayant un côté axial arrière ouvert (512) axialement opposé audit côté axial avant fermé par ladite paroi frontale (513).

11. Bouchon-piston selon la revendication 10, dans lequel ledit volume interne (515) coopère avec un organe d'actionnement d'un dispositif de seringue.

12. Bouchon-piston selon la revendication 11, dans lequel ledit corps cylindrique creux (511) comporte sur sa surface interne un filetage (615) adapté à se visser sur un filet dudit organe d'actionnement.

13. Bouchon-piston selon l'une quelconque des revendications précédentes, dans lequel ladite surface externe axiale frontale (631) est conique et définit une pointe axiale centrale (1810).

14. Bouchon-piston selon l'une quelconque des revendications précédentes, dans lequel ledit profil de déformation (633) est de forme arrondie, de sorte que ladite surface externe axiale frontale (631) de ladite paroi frontale (513) est dépourvue d'angles vifs.

## Patentansprüche

1. Kolbenstopfen (510), der einen zylindrischen Körper (511) aufweist, der eine durch eine Stirnwand (513) geschlossene axial vordere Seite besitzt, wobei der Kolbenstopfen (510) eine äußere Oberfläche aufweist, die mit wenigstens einem sekundären Dichtungsprofil (610) versehen ist, wobei die Stirnwand (513) eine axial vordere äußere Oberfläche (631) aufweist, die mit einer Beschichtung (635), vorteilhaft mit einem Ethylen-Tetrafluorethylen-Film, versehen ist, wobei die Stirnwand (513) an ihrem radial äußeren Rand ein primäres Dichtungsprofil (630) aufweist, **dadurch gekennzeichnet, dass** die Stirnwand (513) und der zylindrische Körper (511) aus unterschiedlichen Materialien verwirklicht sind, wobei der hohle zylindrische Körper (511) an der Stirnwand (513) konfektioniert ist, und dass die axial vordere äußere Oberfläche (631) der Stirnwand (513) ein Verformungsprofil (633) aufweist, das radial innerhalb des primären Dichtungsprofils (630) der Stirnwand (513) angeordnet ist, wobei das Verformungsprofil (633) dafür ausgelegt ist, sich radial zu verformen, um das Einsetzen und/oder das Gleiten des Kolbenstopfens (510) in einen Spritzenkörper zu erleichtern.

2. Kolbenstopfen nach Anspruch 1, wobei der zylindrische Körper (511) aus einem Kautschukmaterial verwirklicht ist, das ein oder mehrere synthetische Polymer-Elastomere enthält, etwa Butyl-Kautschuk, Chlorbutyl-Kautschuk, Brombutyl-Kautschuk, Isobutylen-Kautschuk- und Paramethylstyrol-Copolymere, Isopren-Kautschuk, SBR-Kautschuk, NBR und EPDM.

3. Kolbenstopfen nach Anspruch 2, wobei das Material wenigstens einen Beschleuniger und/oder Füllstoff enthält.

4. Kolbenstopfen nach einem der vorhergehenden Ansprüche, wobei die Stirnwand (513) aus einem Kautschuk-Material verwirklicht ist, das ein oder mehrere synthetische Polymer-Elastomere enthält, etwa Butyl-Kautschuk, Chlorbutyl-Kautschuk, Brombutyl-Kautschuk, Isobutylen-Kautschuk- und Paramethylstyrol-Copolymere, Isopren-Kautschuk, SBR-Kautschuk, NBR und EPDM.

5. Kolbenstopfen nach Anspruch 4, wobei das Material wenigstens einen Beschleuniger und/oder Füllstoff enthält.

6. Kolbenstopfen nach einem der Ansprüche 3 und 5, wobei der wenigstens ein Beschleuniger und/oder Füllstoff des zylindrischen Körpers (511) hinsichtlich Menge und/oder Zusammensetzung von dem wenigstens einen Beschleuniger und/oder Füllstoff der Stirnwand (513) verschieden sind.

7. Kolbenstopfen nach einem der vorhergehenden Ansprüche, wobei der zylindrische Körper (511) ein sekundäres Dichtungsprofil (610) aufweist und die Stirnwand (513) ein sekundäres Dichtungsprofil (610) aufweist.

8. Kolbenstopfen nach Anspruch 7, wobei die sekundären Dichtungsprofile (610) radial vorstehendem Wulste sind.

9. Kolbenstopfen nach einem der vorhergehenden Ansprüche, wobei das Verformungsprofil (633) in Form einer Umfangsrille verwirklicht ist, die in der axial vorderen äußeren Oberfläche (631) der Stirnwand (513) ausgespart ist, wobei die Rille vorteilhaft die Form eines V oder U hat.

10. Kolbenstopfen nach einem der vorhergehenden Ansprüche, wobei der zylindrische Körper (511) hohl ist und ein Innenvolumen (515) definiert, wobei der hohle zylindrische Körper (511) eine offene axial hintere Seite (512) aufweist, die der durch die Stirnwand (513) verschlossenen axial vorderen Seite axial gegenüberliegt.

11. Kolbenstopfen nach Anspruch 10, wobei das Innenvolumen (515) mit einem Betätigungsorgan einer Spritzenvorrichtung zusammenwirkt.

12. Kolbenstopfen nach Anspruch 11, wobei der hohle zylindrische Körper (511) auf seiner inneren Oberfläche ein Gewinde (615) aufweist, das dafür ausgelegt ist, auf ein Gewinde des Betätigungsorgans geschraubt zu werden.

13. Kolbenstopfen nach einem der vorhergehenden Ansprüche, wobei die axial vordere äußere Oberfläche (631) konisch ist und eine mittige axiale Spitze (1810) definiert.

14. Kolbenstopfen nach einem der vorhergehenden Ansprüche, wobei das Verformungsprofil (633) eine abgerundete Form besitzt, derart, dass die axial vordere äußere Oberfläche (631) der Stirnwand (513) keine scharfen Ecken besitzt.

## Claims

1. A stopper/piston (510) comprising a cylindrical body (511) having a front axial end that is closed by a front wall (513), said stopper/piston (510) including an outside surface that is provided with at least one secondary sealing profile (610), said front wall (513) including a front axial outside surface (631) that is provided with a coating (635), advantageously a film made of ethylene tetrafluoroethylene, said front wall (513) including a primary sealing profile (630) on its radially-outer edge, the stopper/piston being **characterized in that** said front wall (513), and said cylindrical body (511) are made of different materials, said hollow cylindrical body (511) being overmolded on said front wall (513), and **in that** said front axial outside surface (631) of said front wall (513) includes a deformation profile (633) that is arranged radially inside said primary sealing profile (630) of said front wall (513), said deformation profile (633) being adapted to deform radially so as to make it easier to insert and/or to slide said stopper/piston (510) into a syringe body.

2. A stopper/piston according to claim 1, wherein said cylindrical body (511) is made of a rubber material, e.g. comprising one or more synthetic elastomeric polymers such as butyl rubber, chlorobutyl rubber, bromobutyl rubber, isobutylene and para-methylstyrene rubber copolymers, isoprene rubber, SBR, NBR, EPDM.

3. A stopper/piston according to claim 2, wherein said material is associated with at least one accelerator and/or filler.

4. A stopper/piston according to any preceding claim, wherein said front wall (513) is made of a rubber material, e.g. comprising one or more synthetic elastomeric polymers such as butyl rubber, chlorobutyl rubber, bromobutyl rubber, isobutylene and para-methylstyrene rubber copolymers, isoprene rubber, SBR, NBR, EPDM.

5. A stopper/piston according to claim 4, wherein said material is associated with at least one accelerator and/or filler.

6. A stopper/piston according to claim 3 and claim 5, wherein said at least one accelerator and/or filler of said cylindrical body (511) is different, in quantity and/or in composition, from said at least one accelerator and/or filler of said front wall (513).

7. A stopper/piston according to any preceding claim, wherein said cylindrical body (511) includes a secondary sealing profile (610), and said front wall (513) includes a secondary sealing profile (610).

8. A stopper/piston according to claim 7, wherein said secondary sealing profiles (610) are radially-projecting beads.

9. A stopper/piston according to any preceding claim, wherein said deformation profile (633) is made in the shape of a peripheral groove that is formed in said front axial outside surface (631) of said front wall (513), said groove advantageously being V-shaped or U-shaped.

10. A stopper/piston according to any preceding claim, wherein said cylindrical body (511) is hollow and defines an internal volume (515), said hollow cylindrical body (511) having an open rear axial end (512) that is axially remote from said front axial end that is closed by said front wall (513).

11. A stopper/piston according to claim 10, wherein said internal volume (515) co-operates with an actuator member of a syringe device.

12. A stopper/piston according to claim 11, wherein, on its inside surface, said hollow cylindrical body (511) includes a screw thread (615) that is adapted to be engaged on a thread of said actuator member.

13. A stopper/piston according to any preceding claim, wherein said front axial outside surface (631) is conical and defines a central axial tip (1810).

14. A stopper/piston according to any preceding claim, wherein said deformation profile (633) is of rounded shape, such that said front axial outside surface (631) of said front wall (513) does not have any sharp angle.
